(19) Europäisches Patentamt
European Patent Office
Office européen des brevets

(11) Veröffentlichungsnummer: **0 626 376 A1**

# (12) EUROPÄISCHE PATENTANMELDUNG

(21) Anmeldenummer: **94107544.2**

(22) Anmeldetag: **16.05.94**

(51) Int. Cl.[5]: **C07D 273/00**, A61K 31/395, C07K 11/00, C07K 11/02

(30) Priorität: **26.05.93 DE 4317432**

(43) Veröffentlichungstag der Anmeldung: **30.11.94 Patentblatt 94/48**

(84) Benannte Vertragsstaaten: **AT BE CH DE DK ES FR GB GR IE IT LI NL SE**

(71) Anmelder: **BAYER AG**

**D-51368 Leverkusen (DE)**

(72) Erfinder: **Scherkenbeck, Dr. Jürgen**
**Auf dem Bruch 49**
**D-51381 Leverkusen (DE)**
Erfinder: **Jeschke, Dr. Peter**
**Heymannstrasse 38**
**D-51373 Leverkusen (DE)**
Erfinder: **Lerchen, Dr. Hans-Georg**
**Morgengraben 14**
**D-51061 Köln (DE)**
Erfinder: **Hagemann, Dr. Hermann**
**Kandinskystrasse 52**
**D-51375 Leverkusen (DE)**
Erfinder: **Harder, Dr. Dr. Achim**
**Europaring 54**
**D-51109 Köln (DE)**
Erfinder: **Mencke, Dr. Norbert**
**Grundermühle 2**
**D-51381 Leverkusen (DE)**
Erfinder: **Plant, Dr. Andrew**
**Im Wiesengrund 16**
**D-51519 Odenthal (DE)**

(54) **Octacyclodepsipeptide mit endoparasitizider Wirkung.**

(57) Gegenstand der vorliegenden Erfindung sind Verbindungen der allgemeinen Formel (I)

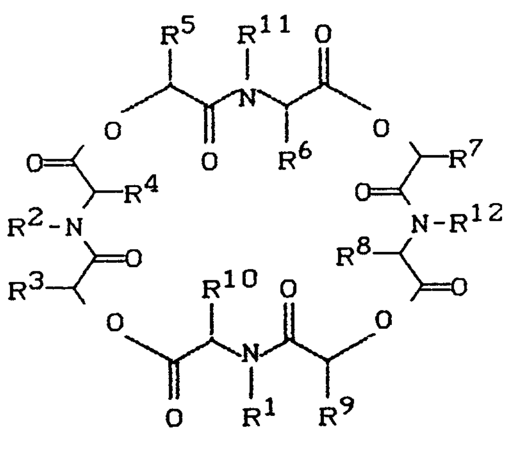

(I)

in welcher

$R^1$ und $R^{12}$ für gleiche oder verschiedene Reste der Gruppe $C_{2-9}$-Alkyl, $C_{1-8}$-Halogenalkyl, $C_{3-6}$-Cycloalkyl, Aralkyl, Aryl stehen,

$R^{11}$ und $R^2$ für gleiche oder verschiedene Reste der Gruppe $C_{1-4}$-Alkyl stehen,

sowie deren Stereoisomere, Verfahren zu ihrer Herstellung und ihre Verwendung als Endoparasitizide.

EP 0 626 376 A1

Die vorliegende Erfindung betrifft neue Octacyclodepsipeptide sowie mehrere Verfahren zu deren Herstellung und ihre Verwendung als Endoparasitizide.

Aus der EP-OS 0 382 173 ist ein cyclisches Depsipeptid mit der Bezeichnung PF 1022 bekannt. Die Verbindung besitzt anthelminthische Wirkung. Bei niedrigen Aufwandmengen läßt die Wirksamkeit allerdings in manchen Fällen zu wünschen übrig.

Gegenstand der vorliegenden Erfindung sind nun:

1. Verbindungen der allgemeinen Formel (I)

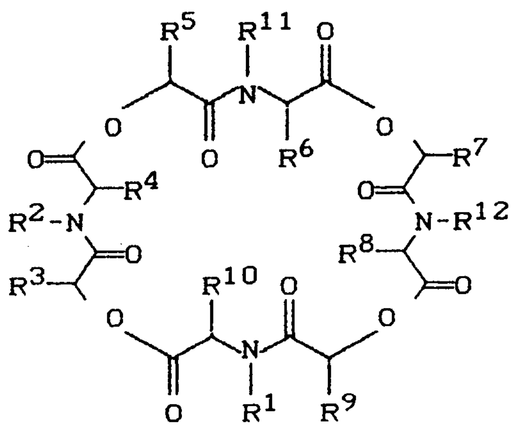

(I)

in welcher

R¹ und R¹² für gleiche oder verschiedene Reste der Gruppe $C_{2-9}$-Alkyl, $C_{1-8}$-Halogenalkyl, $C_{3-6}$-Cycloalkyl, Aralkyl, Aryl stehen,

R³ bis R¹⁰ für gleiche oder verschiedene Reste der Gruppe Wasserstoff, $C_{1-5}$-Alkyl, das gegebenenfalls durch Hydroxy, Alkoxy, Carboxy, Carboxamid, Imidazolyl, Indolyl, Guanidino, Thio- oder Thioalkyl substituiert sein kann, sowie für gegebenenfalls durch Nitro, oder einen Rest $-NR^{13}R^{14}$, wobei $R^{13}$ und $R^{14}$ unabhängig voneinander für Wasserstoff oder Alkyl stehen oder $R^{13}$ und $R^{14}$ gemeinsam mit dem angrenzenden N-Atom für einen carbocyclischen 5-, 6- oder 7-gliedrigen Ring stehen, der gegebenenfalls auch durch O, S und N unterbrochen sein kann und gegebenenfalls durch $C_1$-$C_4$-Alkyl substituiert ist, oder durch Halogen, Hydroxy, Alkyl oder Alkoxy substituiertes Aryl, Alkylaryl und Hetarylmethyl stehen,

R¹¹ und R² für gleiche oder verschiedene Reste der Gruppe $C_{1-4}$-Alkyl stehen,

sowie deren Stereoisomere.

2. Verfahren zur Herstellung der Verbindungen der Formel (I)

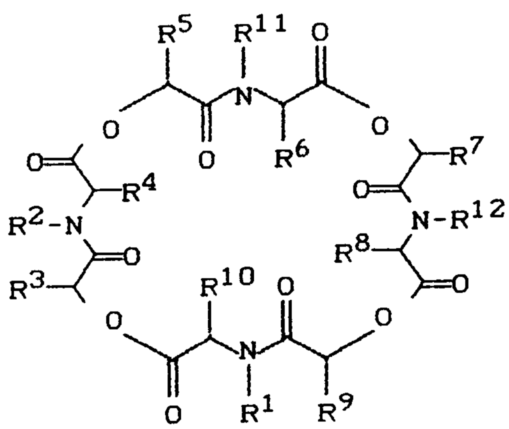

(I)

in welcher

R¹ und R¹² für gleiche oder verschiedene Reste der Gruppe $C_{2-9}$-Alkyl, $C_{1-8}$-Halogenalkyl, $C_{3-6}$-

Cycloalkyl, Aralkyl, Aryl stehen,

$R^3$ bis $R^{10}$ für gleiche oder verschiedene Reste der Gruppe Wasserstoff, $C_{1-5}$-Alkyl, das gegebenenfalls durch Hydroxy, Alkoxy, Carboxy, Carboxamid, Imidazolyl, Indolyl, Guanidino, Thio- oder Thioalkyl substituiert sein kann, sowie für gegebenenfalls durch Nitro, oder einen Rest $-NR^{13}R^{14}$, wobei $R^{13}$ und $R^{14}$ unabhängig voneinander für Wasserstoff oder Alkyl stehen oder $R^{13}$ und $R^{14}$ gemeinsam mit dem angrenzenden N-Atom für einen carbocyclischen 5-, 6- oder 7-gliedrigen Ring stehen, der gegebenenfalls auch durch O, S und N unterbrochen sein kann und gegebenenfalls durch $C_1$-$C_4$ substituiert ist, oder durch Halogen, Hydroxy, Alkyl oder Alkoxy substituiertes Aryl, Alkylaryl und Hetarylmethyl stehen,

$R^{11}$ und $R^2$ für gleiche oder verschiedene Reste der Gruppe $C_{1-4}$-Alkyl stehen,

dadurch gekennzeichnet, daß man offenkettige Octadepsipeptide der Formel (II)

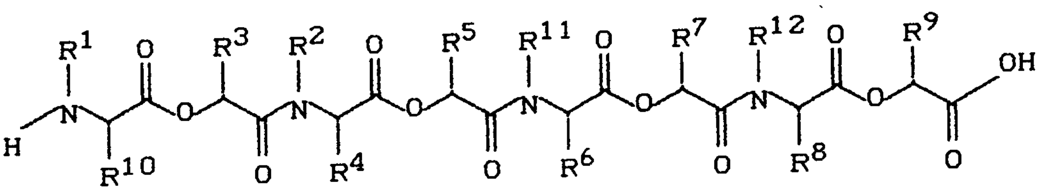

(II)

in welcher

$R^1$ bis $R^{12}$ die oben angegebene Bedeutung haben

in Gegenwart eines Verdünnungsmittels und in Gegenwart eines Kopplungsreagenzes cyclisiert.

3. Offenkettige Octadepsipeptide der Formel (II)

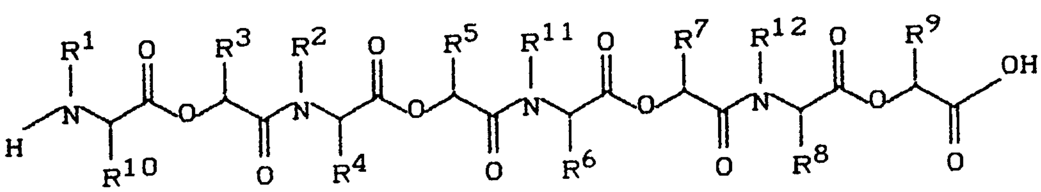

(II)

in welcher

$R^1$ bis $R^{12}$ die oben angegebene Bedeutung haben.

4. Verfahren zur Herstellung der offenkettigen Octadepsipeptide der Formel (II)

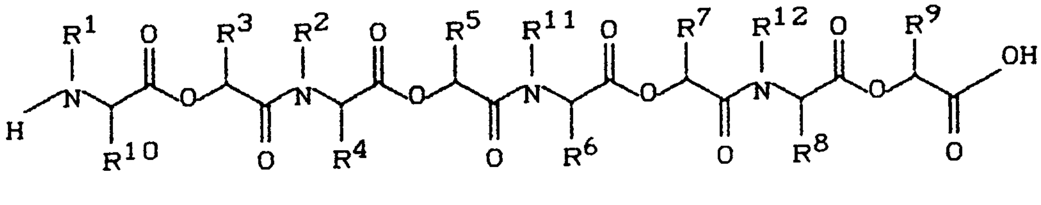

(II)

in welcher

$R^1$ bis $R^{12}$ die oben angegebene Bedeutung besitzen,

dadurch gekennzeichnet, daß man Verbindungen der Formel (III)

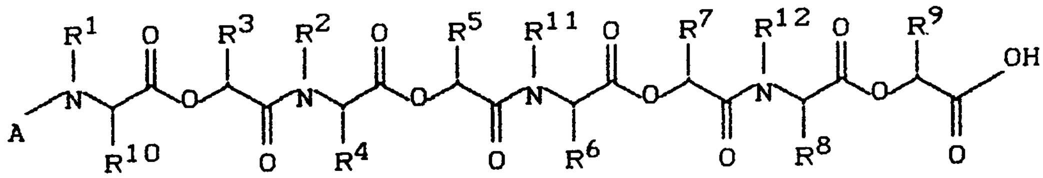

(III)

in welcher

A für Benzyl und

$R^1$ bis $R^{12}$ die oben angegebene Bedeutung besitzen,

in Gegenwart eines Verdünnungsmittels und eines Katalysators hydrogenolysiert.

5. Verbindungen der Formel (III)

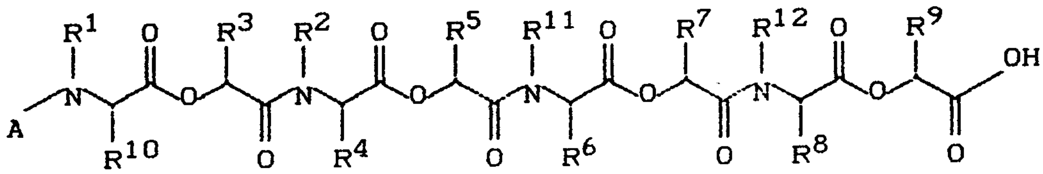

(III)

in welcher

A für Benzyl und

$R^1$ bis $R^{12}$ die oben angegebene Bedeutung besitzen.

6. Verfahren zur Herstellung der Verbindungen der Formel (III)

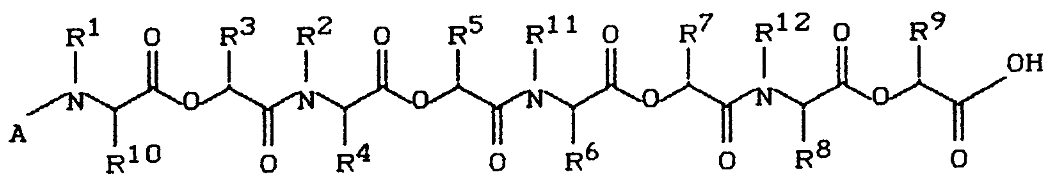

(III)

in welcher

A für Benzyl und

$R^1$ bis $R^{12}$ die oben angegebene Bedeutung besitzen,

dadurch gekennzeichnet, daß man Verbindungen der Formel (IV)

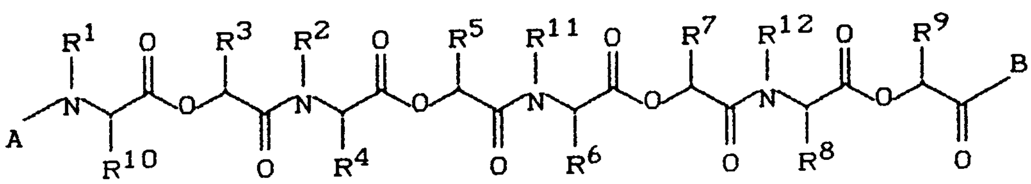

(IV)

in welcher

A für Benzyl und

B für tert.-Butoxy steht sowie

$R^1$ bis $R^{12}$ die oben angegebene Bedeutung besitzen,
in Gegenwart eines Verdünnungsmittels und einer Protonensäure verseift.

7. Verbindungen der Formel (IV)

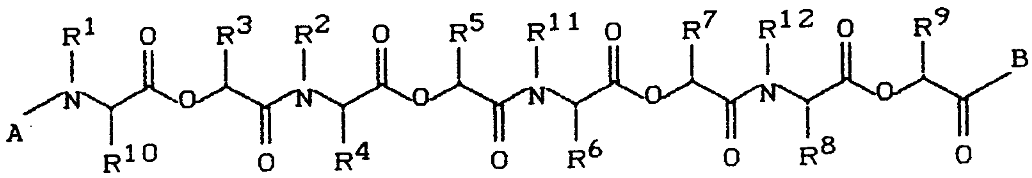

(IV)

in welcher
A für Benzyl und
B für tert.-Butoxy steht und
$R^1$ bis $R^{12}$ die oben angegebene Bedeutung haben.

8. Verfahren zur Herstellung der Verbindungen der Formel (IV)

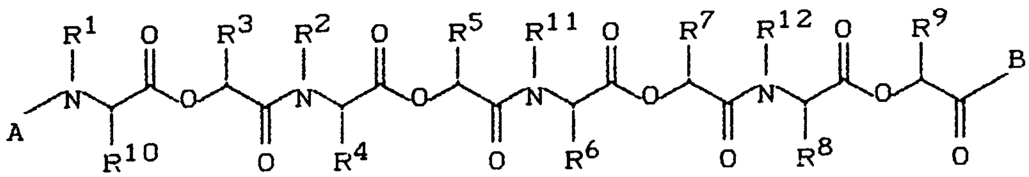

(IV)

in welcher
A für Benzyl und
B für tert.-Butoxy steht und
$R^1$ bis $R^{12}$ die oben angegebene Bedeutung haben,
dadurch gekennzeichnet, daß man Tetradepsipeptide der Formel (V)

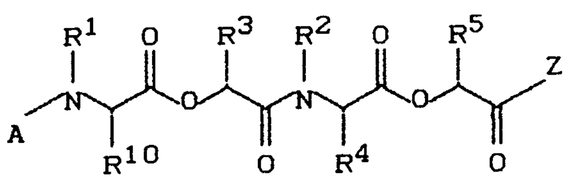

(V)

in welcher
A für Benzyl und
Z für OH oder Cl steht sowie
$R^1$, $R^2$, $R^3$, $R^4$, $R^5$ und $R^{10}$ die oben angegebene Bedeutung haben
und Tetradepsipeptide der Formel (VI)

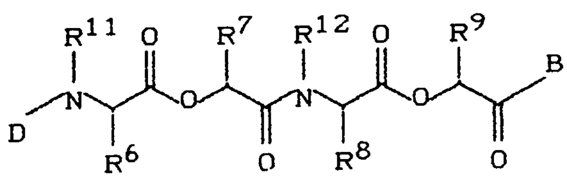

(VI)

in welcher
D für Wasserstoff und
B für tert.-Butoxy steht sowie

$R^6$, $R^7$, $R^8$, $R^9$, $R^{11}$ und $R^{12}$ die oben angegebene Bedeutung haben,
in Gegenwart eines Verdünnungsmittels und eines geeigneten Kopplungsreagenzes kondensiert.

9. Tetradepsipeptide der Formel (V)

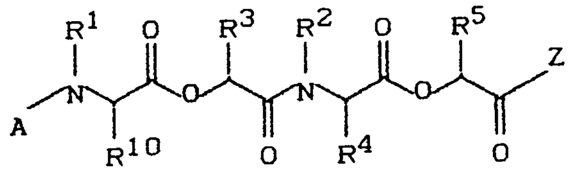

(V)

in welcher

A für Benzyl und
Z für OH oder Cl steht sowie
$R^1$, $R^2$, $R^3$, $R^4$, $R^5$ und $R^{10}$ die oben angegebene Bedeutung haben.

10. Tetradepsipeptide der Formel (VI)

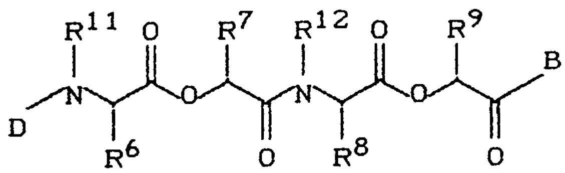

(VI)

in welcher

D für Wasserstoff und
B für tert.-Butoxy steht sowie
$R^6$, $R^7$, $R^8$, $R^9$, $R^{11}$ und $R^{12}$ die oben angegebene Bedeutung besitzen.

11. Verfahren zur Herstellung der Tetradepsipeptide der Formel (V)

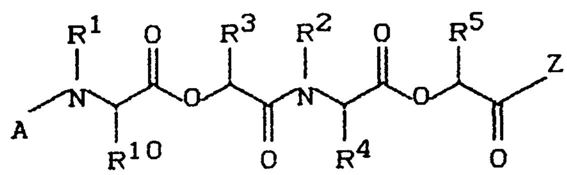

(V)

in welcher

A für Benzyl und
Z für OH oder Cl steht sowie
$R^1$, $R^2$, $R^3$, $R^4$, $R^5$ und $R^{10}$ die oben angegebene Bedeutung haben,
dadurch gekennzeichnet, daß man Tetradepsipeptide der Formel (VII)

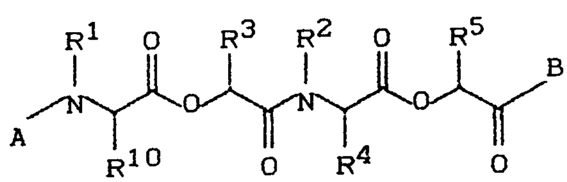

(VII)

in welcher

A für Benzyl und
B für tert.-Butoxy steht sowie
$R^1$, $R^2$, $R^3$, $R^4$, $R^5$ und $R^{10}$ die oben angegebene Bedeutung haben,
in Gegenwart eines Verdünnungsmittels und einer Protonensäure verseift.

12. Verfahren zur Herstellung der Tetradepsipeptide der Formel (VI)

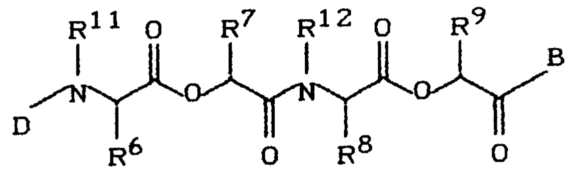

(VI)

in welcher

D für Wasserstoff und

B für tert.-Butoxy steht sowie

$R^6$, $R^7$, $R^8$, $R^9$, $R^{11}$ und $R^{12}$ die oben angegebene Bedeutung haben,

dadurch gekennzeichnet, daß man Tetradepsipeptide Formel (VII)

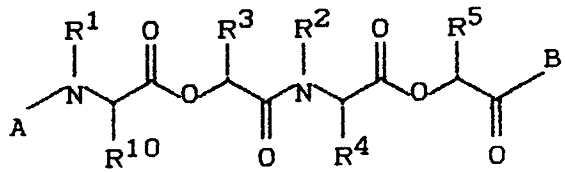

(VII)

in welcher

A für Benzyl und

B für tert.-Butoxy steht sowie

$R^1$, $R^2$, $R^3$, $R^4$, $R^5$ und $R^{10}$ die oben angegebene Bedeutung besitzen,

in Gegenwart eines Verdünnungsmittels und eines Katalysators hydrogenolysiert.

13. Tetradepsipeptide der Formel (VII)

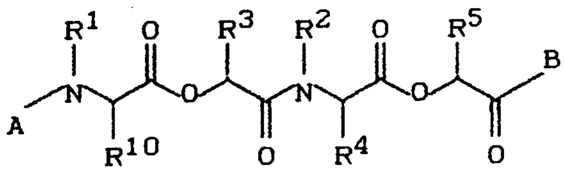

(VII)

in welcher

A für Benzyl und

B für tert.-Butoxy steht sowie

$R^1$, $R^2$, $R^3$, $R^4$, $R^5$ und $R^{10}$ die oben angegebene Bedeutung haben.

14. Verfahren zur Herstellung der Tetradepsipeptide der Formel (VII)

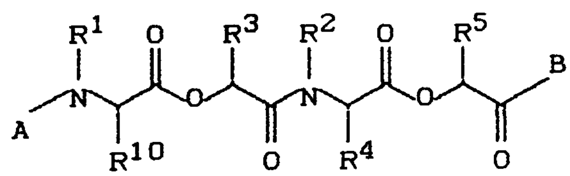

(VII)

in welcher

A für Benzyl und

B für tert.-Butoxy steht sowie

$R^1$, $R^2$, $R^3$, $R^4$, $R^5$ und $R^{10}$ die oben angegebene Bedeutung besitzen,

dadurch gekennzeichnet, daß man Didepsipeptide der Formel (VIII)

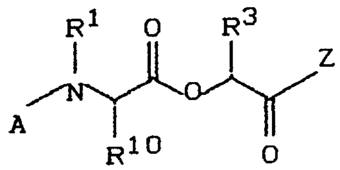

(VIII)

in welcher

A für Benzyl und

Z für OH oder Cl steht sowie

$R^1$, $R^3$ und $R^{10}$ die oben angegebene Bedeutung besitzen und

Didepsipeptide der Formel (IX)

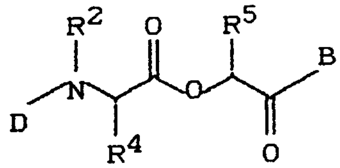

(IX)

in welcher

D für Wasserstoff und

B für tert.-Butoxy steht sowie

$R^2$, $R^4$ und $R^5$ die oben angegebene Bedeutung besitzen,

in einem Verdünnungsmittel in Gegenwart eines geeigneten Kopplungsreagenzes kondensiert.

15. Didepsipeptide der Formel (VIII)

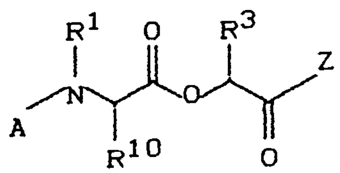

(VIII)

in welcher

A für Benzyl und

Z für OH oder Cl steht sowie

$R^1$, $R^3$ und $R^{10}$ die oben angegebene Bedeutung besitzen.

16. Didepsipeptide der Formel (IX)

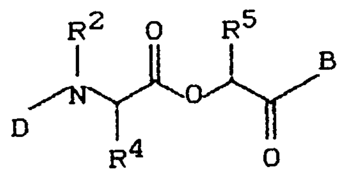

(IX)

in welcher

D für Wasserstoff und

B für tert.-Butoxy steht sowie

$R^2$, $R^4$ und $R^5$ die oben angegebene Bedeutung besitzen.

17. Verfahren zur Herstellung von Didepsipeptiden der Formel (VIII)

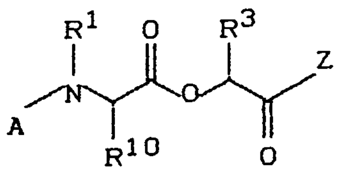

(VIII)

in welcher

Z für OH oder Cl und

A für Benzyl steht sowie

$R^1$, $R^3$ und $R^{10}$ die oben angegebene Bedeutung besitzen. Die Verfahren sind dadurch gekennzeichnet, daß man eine Verbindung der Formel (X)

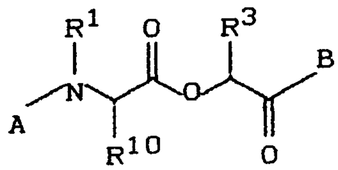

(X)

in welcher

A für Benzyl steht und

B für tert.-Butoxy sowie

$R^1$, $R^3$ und $R^{10}$ die oben angegebene Bedeutung besitzen

und einem Verdünnungsmittel in Gegenwart einer Protonensäure verseift.

18. Verfahren zur Herstellung von Didepsipeptiden der Formel (IX)

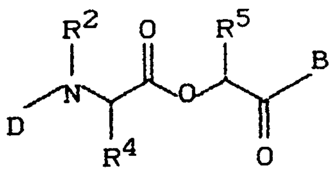

(IX)

in welcher

D für Wasserstoff und

B für tert.-Butoxy steht sowie

$R^2$, $R^4$ und $R^5$ die oben angegebene Bedeutung besitzen,

dadurch gekennzeichnet, daß man Verbindungen der Formel (XI)

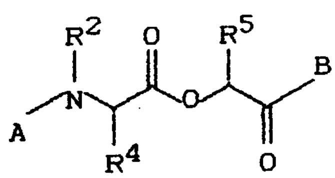

(XI)

in welcher

A für Benzyl und

B für tert.-Butoxy steht sowie

$R^2$, $R^4$ und $R^5$ die oben angegebene Bedeutung besitzen,

in Gegenwart eines Verdünnungsmittels und eines Katalysators hydrogenolysiert.

19. Didepsipeptide der Formel (X)

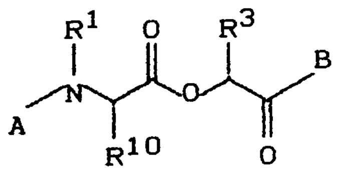

(X)

in welcher

A für Benzyl steht und

B für tert.-Butoxy sowie

$R^1$, $R^3$ und $R^{10}$ die oben angegebene Bedeutung besitzen.

20. Didepsipeptide der Formel (XI)

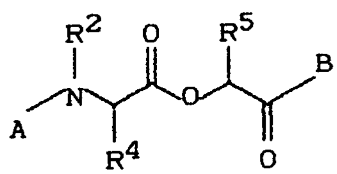

(XI)

in welcher

A für Benzyl und

B für tert.-Butoxy steht sowie

$R^2$, $R^4$ und $R^5$ die oben angegebene Bedeutung besitzen.

21. Verfahren zur Herstellung von Didepsipeptiden der Formel (X)

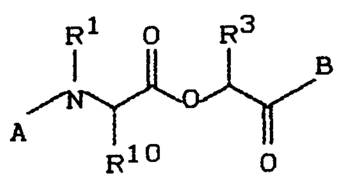

(X)

in welcher

A für Benzyl und

B für tert.-Butoxy steht sowie

$R^1$, $R^3$ und $R^{10}$ die oben angegebene Bedeutung besitzen,

dadurch gekennzeichnet, daß man eine Aminocarbonsäure der Formel (XII)

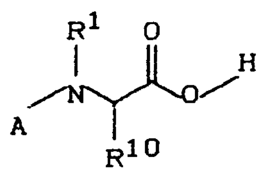

(XII)

in welcher

A für Benzyl und

$R^1$ und $R^{10}$ die oben angegebene Bedeutung besitzen,

in Form ihres Alkalimetallsalzes, bevorzugt ihres Caesiumsalzes, und eine α-Halogencarbonsäure der Formel (XIII)

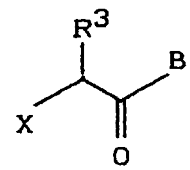

(XIII)

in welcher
X für Cl oder Br und
B für tert.-Butoxy steht sowie
$R^3$ die oben angegebene Bedeutung besitzt,
in Gegenwart eines Verdünnungsmittels koppelt.

22. Verfahren zur Herstellung von Didepsipeptiden der Formel (XI)

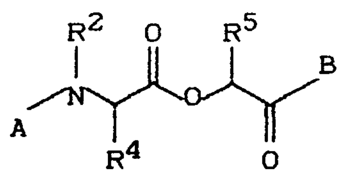

(XI)

in welcher
A für Benzyl und
B für tert.-Butoxy steht sowie
$R^2$, $R^4$ und $R^5$ die oben angegebene Bedeutung besitzen,
dadurch gekennzeichnet, daß man eine Aminocarbonsäure der Formel (XIV)

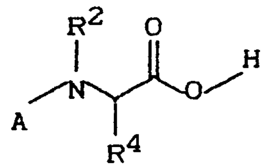

(XIV)

in welcher
A für Benzyl und
$R^1$ und $R^{10}$ die oben angegebene Bedeutung besitzen,
in Form ihres Alkalimetallsalzes, bevorzugt ihres Caesiumsalzes, und eine $\alpha$-Halogencarbonsäure der Formel (XV)

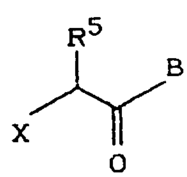

(XV)

in welcher
X für Cl oder Br und
B für tert.-Butoxy steht sowie
$R^5$ die oben angegebene Bedeutung besitzt,
in Gegenwart eines Verdünnungsmittels koppelt.

Schließlich wurde gefunden, daß die neuen Octacyclodepsipeptide der Formel (I) sowie deren Säureadditions-Salze und Metallsalz-Komplexe sehr gute endoparasitizide, insbesondere anthelminthische Eigenschaften besitzen und bevorzugt im Bereich der Veterinärmedizin eingesetzt werden können. Überraschenderweise zeigen die erfindungsgemäßen Stoffe bei der Bekämpfung von Wurmerkrankungen eine deutlich bessere Wirksamkeit als konstitutionell ähnliche, vorbekannte Verbindung gleicher Wirkungsrichtung.

Gegebenenfalls substituiertes Alkyl allein oder als Bestandteil eines Restes in den allgemeinen Formeln bedeutet geradkettiges oder verzweigtes Alkyl mit vorzugsweise 1 bis 9, insbesondere 1 bis 5 Kohlenstoffatomen. Beispielhaft und vorzugsweise seien gegebenenfalls substituiertes Methyl, Ethyl, n- und i-Propyl, n-, i- und t-Butyl, genannt.

Gegebenenfalls substituiertes Alkenyl allein oder als Bestandteil eines Restes in den allgemeinen Formeln bedeutet geradkettiges oder verzweigtes Alkenyl mit vorzugsweise 2 bis 20, insbesondere 2 bis 8 Kohlenstoffatomen. Beispielhaft und vorzugsweise seien gegebenenfalls substituiertes Ethenyl, Propenyl-(1), Propenyl-(2) und Butenyl-(3) genannt.

Gegebenenfalls substituiertes Cycloalkyl in den allgemeinen Formeln bedeutet mono-, bi- und tricyclisches Cycloalkyl mit vorzugsweise 3 bis 10, insbesondere 3, 5 oder 6 Kohlenstoffatomen. Beispielsweise und vorzugsweise seien gegebenenfalls substituiertes Cyclopropyl, Cyclobutyl, Cyclopentyl, Cyclohexyl und Cycloheptyl genannt.

Gegebenenfalls substituiertes Alkoxy in den allgemeinen Formeln bedeutet geradkettiges oder verzweigtes Alkoxy mit vorzugsweise 1 bis 6, insbesondere 1 bis 4 Kohlenstoffatomen. Beispielhaft und vorzugsweise seien gegebenenfalls substituiertes Methoxy, Ethoxy, n- und i-Propoxy und n-, o- und t-Butoxy genannt.

Gegebenenfalls substituiertes Alkylthio in den allgemeinen Formeln bedeutet geradkettiges oder verzweigtes Alkylthio mit vorzugsweise 1 bis 6, insbesondere 1 bis 4 Kohlenstoffatomen. Beispielhaft und vorzugsweise seien gegebenenfalls substituiertes Methylthio, Ethylthio, n-und i-Propylthio, n-, o- und t-Butylthio genannt.

Halogenalkyl in den allgemeinen Formeln enthält 1 bis 4, insbesondere 1 oder 2 Kohlenstoffatome und vorzugsweise 1 bis 9, insbesondere 1 bis 5 gleiche gleiche oder verschiedene Halogenatome, wobei als Halogenatome vorzugsweise Fluor, Chlor und Brom, insbesondere Fluor und Chlor stehen. Beispielhaft seien Trifluormethyl, Chlor-di-fluormethyl, 2,2,2-Trifluorethyl und Pentafluormethyl, Perfluor-t-butyl genannt.

Gegebenenfalls substituiertes Aryl in den allgemeinen Formeln bedeutet vorzugsweise gegebenenfalls substituiertes Phenyl oder Naphthyl, insbesondere Phenyl.

Gegebenenfalls substituiertes Arylalkyl in den allgemeinen Formeln bedeutet gegebenenfalls im Arylteil und/oder Alkylteil substituiertes Aralkyl mit vorzugsweise 6 oder 10, insbesondere 6 Kohlenstoffatomen im Arylteil (vorzugsweise Phenyl oder Naphthyl, insbesondere Phenyl) und vorzugsweise 1 bis 4, insbesondere 1 oder 2 Kohlenstoffatomen im Alkylteil, wobei der Alkylteil geradkettig oder verzweigt sein kann. Beispielhaft und vorzugsweise seien gegebenenfalls substituiertes Benzyl und Phenylethyl genannt.

Gegebenenfalls Heteroaryl allein oder als Bestandteil eines Restes bedeutet in den allgemeinen Formeln 5- bis 7-gliedrige Ringe mit vorzugsweise 1 bis 3, insbesondere 1 oder 2 gleichen oder verschiedenen Heteroatomen. Als Heteroatome stehen Sauerstoff, Schwefel oder Stickstoff. Beispielhaft und vorzugsweise seien gegebenenfalls substituiertes Furyl, Thiophenyl, Pyrazolyl, Imidazolyl, 1,2,3- und 1,2,4-Triazolyl, Oxazolyl, Isoxazolyl, Thiazolyl, Isothiazolyl, 1,2,3-, 1,3,4-, 1,2,4- und 1,2,5-Oxadiazolyl, Azepinyl, Pyrrolyl, Isopyrrolyl, Pyridyl, Piperazinyl, Pyridazinyl, Pyrimidinyl, Pyrazinyl, 1,3,5-, 1,2,4- und 1,2,3-Triazinyl, 1,2,4-, 1,2,3-, 1,3,6- und 1,2,6-Oxazinyl, Oxepinyl, Thiepinyl und 1,2,4-Diazepinyl genannt.

Die gegebenenfalls substituierten Reste der allgemeinen Formeln können einen oder mehrere, vorzugsweise 1 bis 3, insbesondere 1 bis 2 gleiche oder verschiedene Substituenten tragen. Als Substituenten seien beispielhaft und vorzugsweise aufgeführt:

Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Difluormethyl, Trifluormethyl; Hydorxy; Halogen, vorzugsweise Fluor, Chlor, Brom, insbesondere Fluor, Chlor, Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methylethylamino, n- und i-Propylamino und Methyl-n-Butylamino; Acylreste wie Carboxyl; Carboxy mit vorzugsweise 2 bis 4, insbesondere 2 oder 3 Kohlenstoffatomen, wie Carbomethoxy und Carboethoxy; Alkylsulfinyl mit 1 bis 4, insbesondere 1 bis 2 Kohlenstoffatomen, Halogenalkylsulfinyl mit 1 bis 4, insbesondere 1 bis 2 Kohlenstoffatomen und 1 bis 5 Halogenatomen wie Trifluormethylsulfinyl; Sulfonyl ($-SO_3H$); Alkylsulfonyl mit vorzugsweise 1 bis 4, insbesondere 1 oder 2 Kohlenstoffatomen, wie Methylsulfonyl und Ethylsulfonyl; Halogenalkylsulfonyl mit 1 bis 4, insbesondere 1 bis 2 Kohlenstoffatomen und 1 bis 5 Halogenatomen wie Trifluormethylsulfonyl, Perfluor-t,n,s-butylsulfonyl; Arylsulfonyl mit vorzugsweise 6 oder 10 Arylkohlenstoffatomen, wie Phenylsulfonyl; Acyl, Aryl, Aryloxy, Heteroaryl, Heteroaryloxy,

die ihrerseits einen der oben genannten Substituenten tragen können sowie der Formiminorest

$$\begin{array}{l} \;\;H \\ \;\;| \\ -C=N-O-Alkyl. \end{array}$$

Bevorzugt werden Verbindungen der Formel (I) eingesetzt, in welcher

$R^1$ und $R^{12}$ unabhängig voneinander für Ethyl, Propyl, Butyl oder Phenyl, das gegebenenfalls substituiert ist durch Halogen, $C_{1-4}$-Alkyl, OH, $C_{1-4}$-Halogenalkyl, $C_{1-4}$-Alkoxy, $C_{1-4}$-Halogenalkoxy, sowie für $CF_3$, Benzyl oder Phenylethyl stehen, die gegebenenfalls durch die oben angegebenen Reste substituiert sein können;

$R^3$ bis $R^{10}$ unabhängig voneinander für Wasserstoff, $C_{1-5}$-Alkyl, das gegebenenfalls durch $C_{1-4}$-Alkoxy, Carboxamid, Imidazolyl, Indolyl, Thio und $C_{1-4}$-Thioalkyl, sowie gegebenenfalls durch Nitro, oder einen Rest $-NR^{13}R^{14}$, wobei $R^{13}$ und $R^{14}$ unabhängig voneinander für Wasserstoff oder Alkyl stehen oder $R^{13}$ und $R^{14}$ gemeinsam mit dem angrenzenden N-Atom für einen carbocyclischen 5-, 6- oder 7-gliedrigen Ring stehen, der gegebenenfalls auch durch O, S und N unterbrochen sein kann und gegebenenfalls durch $C_1$-$C_4$-Alkyl substituiert ist, oder durch Halogen und $C_{1-4}$-Alkoxy substituiertes Phenyl, Benzyl, Phenylethyl oder Hetarylmethyl stehen,

$R^2$ und $R^{11}$ unabhängig voneinander für Methyl, Ethyl, iso-Propyl, Propyl stehen.

Besonders bevorzugt sind Verbindungen der Formel (I), in welcher

$R^1$ und $R^{12}$ unabhängig voneinander für Ethyl, Propyl, iso Propyl, Butyl oder Phenyl stehen,

$R^3$ bis $R^{10}$ unabhängig voneinander für Wasserstoff, $C_{1-5}$-Alkyl, das gegebenenfalls durch Methoxy, Ethoxy, Imidazolyl, Indolyl, Guanidino oder Methylthio, Ethylthio substituiert sein kann, sowie gegebenenfalls durch Nitro, oder einen Rest $-NR^{13}R^{14}$, wobei $R^{13}$ und $R^{14}$ unabhängig voneinander für Wasserstoff oder Alkyl stehen oder $R^{13}$ und $R^{14}$ gemeinsam mit dem angrenzenden N-Atom für einen carbocyclischen 5-, 6- oder 7-gliedrigen Ring stehen, der gegebenenfalls auch durch O, S und N unterbrochen sein kann und gegebenenfalls durch $C_1$-$C_4$-Alkyl substituiert ist, oder durch Halogen und $C_{1-4}$-Alkoxy substituiertes Phenyl, Benzyl, Phenylethyl oder Hetarylmethyl stehen,

$R^2$ und $R^{11}$ unabhängig voneinander für Methyl, Ethyl, Propyl oder Butyl stehen.

Bei Verfahren 2 werden Octadepsipeptide in Gegenwart von Verdünnungsmitteln und geeigneten Kopplungsreagenzien cyclisiert.

Als Kopplungsreagenzien eignen sich alle Verbindungen die zur Knüpfung einer Amidbindung geeignet sind (vgl. z.B.: Houben-Weyl, Methoden der organischen Chemie, Band 15/2; Bodenzky et al., Peptide Synthesis 2nd ed., Wiley u. Sons, New York 1976).

Vorzugsweise kommen folgende Methoden in Frage, Aktivestermethode mit Pentafluorphenol (PfP), N-Hydroxysuccinimid, 1-Hydroxybenzotriazol, Kopplung mit Carbodiimiden wie Dicyclohexylcarbodiimid oder N'-(3-Dimethylaminopropyl)-N-ethylcarbodiimid (EBC) sowie die Gemischte-Anhydrid-Methode oder die Kopplung mit Phosphoniumreagenzien, wie Benzotriazol-1-yl-oxy-tris(dimethylaminophosphonium)-hexafluorphosphat (BOP), Bis-(2-oxo-3-oxazolidinyl)-phosphoniumsäurechlorid (BOP-Cl) oder mit Phosphonsäureesterreagenzien wie Cyanphosphonsäurediethylester (DEPC) und Diphenylphosphorylazid (DPPA).

Besonders bevorzugt ist die Kopplung mit Bis(2-oxo-3-oxazolidinyl)-phosphoniumsäurechlorid (BOP-Cl) und N'-(3-Dimethylaminopropyl)-N-ethylcarbodiimid (EDC) in Gegenwart von 1-Hydroxybenzotriazol (HOBt).

Die Umsetzung erfolgt bei Temperaturen von 0 - 150 °C, bevorzugt bei 20 - 100 °C, besonders bevorzugt bei Raumtemperatur.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Die Cyclisierung wird in Gegenwart einer Base durchgeführt.

Als Basen kommen anorganische und organische Basen in Frage. Als Basen seien genannt Alkali- und Erdalkalihydroxide, -carbonate, -hydrogencarbonate, -alkoholate, ferner Amine wie insbesondere tertiäre Amine z.B. Trimethylamin, Triethylamin, N-Methylmorpholin, Pyridin, Picoline, N-Ethylpyrrolidin, Diazabicyclo(4,3,0)-undecen(DBU), 1,4-Diazabicyclo(2,2,2)octan (DABCO), Diazabicyclo(3,2,0)nonen (DBN), Ethyl-diisopropylamin.

Die Verbindungen der Formeln (II) und die Basen werden im Verhältnis 1:1 bis 1:1,5 zueinander eingesetzt. Bevorzugt ist ein etwa äquimolares Verhältnis.

Nach erfolgter Umsetzung wird das Verdünnungsmittel abdestilliert und die Verbindungen der Formel (I) in üblicher Weise, z.B. chromatographisch, gereinigt.

Die Reaktion gemäß Verfahren 4 wird unter Verwendung von Hydrierungsmitteln durchgeführt.

Als bevorzugtes Hydrierungsmittel sei Wasserstoff in Gegenwart der üblichen Hydrierungskatalysatoren, wie z.B. Raney-Nickel, Palladium und Platin genannt.

Das Verfahren wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Methyl-tert.-butylether, Glykoldimethylether und Diglykoldimethylether, Tetrahydrofuran und Dioxan, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid; ferner auch Alkohole wie Methanol, Ethanol, Propanol, Isopropanol, Butanol, Isobutanol, sec.-Butanol, tert.-Butanol, Pentanol, Isopentanol, sec.-Pentanol und tert.-Pentanol sowie auch Wasser.

Die Reaktionstemperaturen können bei dem erfindungsgemäßen Verfahren in einem größeren Bereich variiert werden. Im allgemeinen arbeitet man bei Temperaturen zwischen -20 °C und +200 °C, vorzugsweise bei Temperaturen zwischen 0 °C und 120 °C.

Das erfindungsgemäße Verfahren wird im allgemeinen unter Normaldruck durchgeführt. Es ist jedoch auch möglich, unter erhöhtem, im allgemeinen zwischen 10 und 100 bar, zu arbeiten.

Die Reaktion gemäß Verfahren 6 wird vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuertriamid.

Die Reaktion wird in Gegenwart von anorganischen oder organischen Protonensäuren durchgeführt. Als solche seien z.B. genannt: Salzsäure, Schwefelsäure, Trifluoressigsäure, Essigsäure, Ameisensäure.

Die Umsetzung erfolgt bei Temperaturen zwischen -20 und +50 °C, vorzugsweise zwischen -10 und +20 °C bei Normaldruck oder erhöhtem Druck. Es wird bevorzugt bei Normaldruck gearbeitet.

Die Reaktion gemäß Verfahren 8 wird vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuertriamid.

Die Reaktion wird in Gegenwart von anorganischen oder organischen Saureakzeptoren durchgeführt.

Als solche seien z.B. genannt:
Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kaliummethylat bzw. -ethylat, ferner aliphatische, aromatische oder heterocycllsche Amine, beispielsweise Triethylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO), Ethyl-diisopropylamin.

Die Umsetzung erfolgt bei Temperaturen zwischen 10 und 150 °C, vorzugsweise zwischen 20 bis 100 °C bei Normaldruck oder erhöhtem Druck. Es wird bevorzugt bei Normaldruck gearbeitet.

Verfahren 11 wird durchgeführt, wie weiter oben bei der Durchführung von Verfahren 6 angegeben.

Das oben unter 12 beschriebene erfindungsgemäße Verfahren wird wie bei Verfahren 4 angegeben durchgeführt.

Verfahren 14 wird durchgeführt wie weiter oben bei der Durchführung von Verfahren 8 angegeben.

Verfahren 17 wird durchgeführt wie weiter oben bei der Durchführung von Verfahren 6 angegeben.

Verfahren 18 wird durchgeführt wie weiter oben bei der Durchführung von Verfahren 12 angegeben.

Das erfindungsgemäße Verfahren 21 wird in Gegenwart eines Metallcarbonats durchgeführt, oder die Aminosäuren der Formel (XII) werden in Form ihrer Salze, bevorzugt Alkalisalze, besonders bevorzugt ihrer Caesiumsalze, eingesetzt. Vorzugsweise werden Alkalimetall- oder Erdalkalimetallcarbonate, wie Lithium-, Natrium-, Kalium-, Magnesium- und Calcium-Carbonat, eingesetzt.

Das erfindungsgemäße Verfahren 21 wird unter Verwendung eines aprotisch polaren organischen Lösungsmittels durchgeführt. Hierzu gehören vorzugsweise Ether wie Diethylether, Dipropylether, Diisopropylether, Dibutylether, Diisobutylether, Tetrahydrofuran und Dioxan, Ketone wie Aceton, Methylethylketon, Diethylketon, Methylisopropylketon und Methylisobutylketon, Ester wie Essigsäuremethylester und Essigsäureethylester, Nitrile wie Acetonitril und Propionitril, Amide wie Dimethylformamid und Dimethylacetamid, ferner N-Methyl-pyrrolidon, Dimethylsulfoxid, Tetramethylensulfon und Hexamethylphosphorsäuretriamid.

Die Reaktionstemperaturen können bei dem erfindungsgemäßen Verfahren 21 in einem größeren Bereich variiert werden. Im allgemeinen arbeitet man bei Temperaturen zwischen -20 °C und +30 °C, vorzugsweise bei Temperaturen zwischen -5 °C und +10 °C.

Das erfindungsgemäße Verfahren 21 wird im allgemeinen unter Normaldruck durchgeführt. Es ist jedoch auch möglich, unter erhöhtem oder vermindertem Druck im allgemeinen zwischen 0,1 und 10 bar, zu arbeiten.

Verfahren 22 wird durchgeführt wie bei Verfahren 21 angegeben.

Die Wirkstoffe eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen insbesondere:
Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., protostrongylus spp., Neostrongylus spp., Cystocaulus spp.,

Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; Halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugofügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß.Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsauren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8$/$C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$,Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:

Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykol ether;

ampholytische Tenside wie Di-Na-N-lauryl-$\beta$-iminodipropionat oder Lecithin;

anionaktive Tenside, wie Na-Laurylsulfat, Fettalkoholethersulfate, Mono/Dialkylpolyglykoletherorthophosphorsäureester-monoethanolaminsalz;

kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert. Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 5 bis 50 Gewichtsprozent.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

## Beispiel A

In vivo Nematodentest

Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzeiren (Dosis effectiva).

Geprüfte Wirkstoffe und wirksame Dosierungen (Dosis effectiva) sind aus der nachfolgenden Tabelle ersichtlich:

| Wirkstoff Beispiel Nr. | Dosis effectiva in mg/kg |
|---|---|
| 1 | 5 |
| 2 | 5 |
| 3 | 5 |

Die Herstellung der erfindungsgemäßen Wirkstoffe geht aus den nachfolgenden Beispielen hervor.

Herstellungsbeispiele

1. Herstellung der Verbindungen der Formel (I) gemäß Verfahren 2

Zu einer Lösung der Verbindung II (0,104 mmol) und Hünig-Base (0,258 mmol) in Dichlormethan (100 ml) wurde bei 0°C BOP-Cl (0,124 mmol) zugegeben und 24 Stunden bei Zimmertemperatur nachgerührt. Nach dieser Zeit wurden dieselben Mengen BOP-Cl und Base zugesetzt und weitere 24 Stunden gerührt. Die Lösung wurde zweimal mit gesättigter Natriumhydrogencarbonatlösung gewaschen, über Natriumsulfat getrocknet und eingeeingt. Der Rückstand wurde säulenchromatographisch mit dem Laufmittel Cyclohexan-Ethylacetat 2:1 gereinigt.

Es wurden Verbindungen der Formel (I) erhalten, in welcher die Substituenten die folgende Bedeutung haben:

Tabelle 1

| Bsp. Nr. | $R^1$ | $R^{12}$ | $R^9$ | $R^8$ | $R^7$ | $R^6$ | $R^5$ | $R^4$ | $R^3$ | $R^{10}$ | $R^{11}$ | $R^2$ | FAB-MS m/Z (%) |
|---|---|---|---|---|---|---|---|---|---|---|---|---|---|
| 1 | Et | Et | Me | i-Bu | Bn | i-Bu | Me | i-Bu | Bn | i-Bu | Et | Et | 1027 (100, $(M+Na)^+$), 1005 (14, $(M+H)^+$) |
| 2 | Propyl | Propyl | " | " | " | " | " | " | " | " | Propyl | Propyl | 1083 (100, $(M+Na)^+$), 1061 (64, $(M+H)^+$) |
| 3 | i-Propyl | i-Propyl | " | " | " | " | " | " | " | " | i-Propyl | i-Propyl | 1061 (45, $(M+H)^+$) |
| 4 | Phenyl | Phenyl | " | " | " | " | " | " | " | " | Me | Me | |
| 5 | Et | Et | " | " | " | " | " | " | " | " | Me | Me | |
| 6 | Propyl | Propyl | " | " | " | " | " | " | " | " | Me | Me | |
| 7 | Et | Et | " | " | 2-Cl-Bn | " | " | " | 2-Cl-Bn | " | Me | Me | |
| 8 | Et | Et | " | " | 3-Cl-Bn | " | " | " | 3-Cl-Bn | " | Et | Et | |
| 9 | Propyl | i-Propyl | " | " | 4-Cl-Bn | " | " | " | 4-Cl-Bn | " | Propyl | i-Propyl | |
| 10 | Propyl | i-Propyl | " | " | Bn | " | " | " | Bn | " | Propyl | i-Propyl | |

Me = Methyl
Et = Ethyl
Bu = Butyl
Bn = Benzyl

2. Herstellung der Verbindungen der Formel (II) gemäß Verfahren 4.

Eine Lösung einer Verbindung der Formel III (1,222 mmol) in Ethanol (50 ml) wurde in Gegenwart von $Pd(OH)_2/C$ (20 %; 200 mg) bis zur Beendigung der Wasserstoffaufnahme hydriert (ca. 2 Stunden). Nach Abfiltrieren des Katalysators fiel 92 % Verbindung der Formel (II) an, die ohne zusätzliche Reinigung weiter umgesetzt wurde.

Gemäß dieser Vorschrift wurden Verbindungen der Formel (II) erhalten, in welcher die Substituenten die folgende Bedeutung haben:

Tabelle 2

| Bsp. Nr. | $R^1$ | $R^{12}$ | $R^9$ | $R^8$ | $R^7$ | $R^6$ | $R^5$ | $R^4$ | $R^3$ | $R^{10}$ | $R^{11}$ | $R^2$ |
|---|---|---|---|---|---|---|---|---|---|---|---|---|
| 1 | Et | Et | Me | i-Bu | Bn | i-Bu | Me | i-Bu | Bn | i-Bu | Et | Et |
| 2 | Propyl | Propyl | " | " | " | " | " | " | " | " | Propyl | Propyl |
| 3 | i-Propyl | i-Propyl | " | " | " | " | " | " | " | " | i-Propyl | i-Propyl |
| 4 | Me | Me | " | s-Bu | " | s-Bu | " | s-Bu | " | s-Bu | Me | Me |
| 5 | Me | Me | " | i-Pr | " | i-Pr | " | i-Pr | " | i-Pr | Me | Me |
| 6 | Me | Me | " | Bn | " | Bn | " | Bn | " | Bn | Me | Me |
| 7 | Me | Me | " | i-Bu | 2-Cl-Bn | i-Bu | " | i-Bu | 2-Cl-Bn | i-Bu | Me | Me |
| 8 | Me | Me | " | " | 3-Cl-Bn | " | " | " | 3-Cl-Bn | " | Me | Me |
| 9 | Me | Me | " | " | 4-Cl-Bn | " | " | " | 4-Cl-Bn | " | Me | Me |
| 10 | Propyl | i-Propyl | " | " | Bn | " | " | " | Bn | " | Propyl | i-Propyl |

Me = Methyl
Et = Ethyl
Bu = Butyl
Py = Propyl
Bn = Benzyl

3. Herstellung der Verbindungen der Formel (III) gemäß Verfahren 6

In eine Lösung des tert.-Butylesters der Formel (IV) (1.609 mmol) in Dichlormethan (40 ml) wurde bei 0 °C 1,5 h HCl-Gas eingeleitet. Anschließend wurde auf Raumtemperatur aufgewärmt und 12 h nachgerührt. Die Lösung wurde einrotiert und im Hochvakuum getrocknet. Der Rückstand wurde ohne weitere Reinigung umgesetzt.

Gemäß dieser Vorschrift wurden Verbindungen der Formel (III) erhalten, in welcher die Substituenten die folgende Bedeutung haben:

Tabelle 3

| Nr. | $R^1$ | $R^{12}$ | $R^9$ | $R^8$ | $R^7$ | $R^6$ | $R^5$ | $R^4$ | $R^3$ | $R^{10}$ | $R^{11}$ | $R^2$ | A | B |
|---|---|---|---|---|---|---|---|---|---|---|---|---|---|---|
| 21 | Et | Et | Me | i-Bu | Bn | i-Bu | Me | i-Bu | Bn | i-Bu | Et | Et | Bn | OH |
| 22 | Propyl | Propyl | " | " | " | " | " | " | " | " | Propyl | Propyl | " | " |
| 23 | i-Propyl | i-Propyl | " | " | " | " | " | " | " | " | i-Propyl | i-Propyl | " | " |
| 24 | Phenyl | Phenyl | " | " | " | " | " | " | " | " | Me | Me | " | " |
| 25 | Et | Et | " | " | " | " | " | " | " | " | Me | Me | " | " |
| 26 | Propyl | Propyl | " | " | " | " | " | " | " | " | Me | Me | " | " |
| 27 | i-Propyl | i-Propyl | " | " | " | " | " | " | " | " | Me | Me | " | " |
| 28 | Et | Propyl | " | " | " | " | " | " | " | " | Et | Propyl | " | " |
| 29 | Et | i-Propyl | " | " | " | " | " | " | " | " | Et | i-Propyl | " | " |
| 30 | Propyl | i-Propyl | " | " | " | " | " | " | " | " | Propyl | i-Propyl | " | " |

4. Herstellung der Verbindungen der Formel (IV) gemäß Verfahren 8
Tetradepsipeptide der Formel (VI) (2,52 mmol) und der Formel V (2,52 mmol) wurden in Dichlormethan (15 ml) vorgelegt und die auf 0 ° C gekühlte Lösung mit Ethyldiisopropylamin (0,912 mmol) und BOP-Cl (0,438 mmol) versetzt. Es wurde 1 Stunde bei 0 ° C und 1,5 Stunden bei Raumtemperatur nachgerührt, dann mit Dichlormethan verdünnt, zweimal mit wenig Wasser gewaschen, über Natriumsulfat getrocknet und eingeengt. Der Rückstand wurde an Kieselgel mit dem Laufmittel Cyclohexan-BuOMe = 2:1 gereinigt.

Gemäß dieser Vorschrift wurden Verbindungen der Formel (IV) erhalten, in welcher die Substituenten die folgende Bedeutung haben.

Tabelle 4

| Nr. | $R^1$ | $R^{12}$ | $R^9$ | $R^8$ | $R^7$ | $R^6$ | $R^5$ | $R^4$ | $R^3$ | $R^{10}$ | $R^{11}$ | $R^2$ | A | B |
|---|---|---|---|---|---|---|---|---|---|---|---|---|---|---|
| 31 | Et | Et | Me | i-Bu | Bn | i-Bu | Me | i-Bu | Bn | i-Bu | Et | Et | Bn | t-BuO |
| 32 | Propyl | Propyl | " | " | " | " | " | " | " | " | Propyl | Propyl | " | " |
| 33 | i-Propyl | i-Propyl | " | " | " | " | " | " | " | " | i-Propyl | i-Propyl | " | " |
| 34 | Phenyl | Phenyl | " | " | " | " | " | " | " | " | Me | Me | " | " |
| 35 | Et | Et | " | " | " | " | " | " | " | " | Me | Me | " | " |
| 36 | Propyl | Propyl | " | " | " | " | " | " | " | " | Me | Me | " | " |
| 37 | i-Propyl | i-Propyl | " | " | " | " | " | " | " | " | Me | Me | " | " |
| 38 | Et | Propyl | " | " | " | " | " | " | " | " | Et | Propyl | " | " |
| 39 | Et | i-Propyl | " | " | " | " | " | " | " | " | Et | i-Propyl | " | " |
| 40 | Propyl | i-Propyl | " | " | " | " | " | " | " | " | Propyl | i-Propyl | " | " |

5. Herstellung der Verbindungen der Formel (V) gemäß Verfahren 11

In eine Lösung des Tetradepsipeptids mit der Formel (VII) (2.848 mmol) in Dichlormethan (50 ml) wurde bei 0 °C HCl-Gas 2 h eingeleitet.

Anschließend wurde 8 Stunden bei Raumtemperatur nachgerührt, eingeengt und im Hochvakuum getrocknet. Der Rückstand wurde ohne weitere Reinigung eingesetzt.

Gemäß dieser Vorschrift wurden die folgenden Verbindungen der Formel (V) erhalten, in welcher die Substituenten die folgende Bedeutung haben:

Tabelle 5

| Nr. | $R^1$ | $R^{12}$ | $R^9$ | $R^8$ | $R^7$ | $R^{10}$ | A | Z |
|---|---|---|---|---|---|---|---|---|
| 41 | Et | Et | Me | i-Bu | Bn | i-Bu | Bn | OH |
| 42 | Propyl | Propyl | " | " | " | " | " | " |
| 43 | i-Propyl | i-Propyl | " | " | " | " | " | " |
| 44 | Phenyl | Phenyl | " | " | " | " | " | " |
| 45 | Et | Propyl | " | " | " | " | " | " |
| 46 | Et | i-Propyl | " | " | " | " | " | " |
| 47 | Propyl | i-Propyl | " | " | " | " | " | " |

6. Herstellung der Verbindungen der Formel (VI) gemäß Verfahren 12

Tetradepsipeptide der Formel (VII) (9,53 mmol) wurden in Ethanol (37 ml) aufgelöst, mit 0,6 g $Pd(OH)_2/C$ (20 %) versetzt und bis zur Beendigung der Wasserstoffaufnahme bei Raumtemperatur hydriert. Nach Abfiltrieren des Katalysators und Einengen des Lösungsmittels wurde der Rückstand an Kieselgel mit dem Laufmittel BuOMe-Cyclohexan-Ethanol = 1:1:0,5 säulenchromatographisch getrennt.

Analog wurden die Verbindungen der Formel (VI) erhalten, in welcher die Substituenten die folgende Bedeutung haben:

Tabelle 6

| Nr. | $R^1$ | $R^{12}$ | $R^9$ | $R^8$ | $R^7$ | $R^{10}$ | D | B |
|---|---|---|---|---|---|---|---|---|
| 48 | Et | Et | Me | i-Bu | Bn | i-Bu | H | O t-Bu |
| 49 | Propyl | Propyl | " | " | " | " | " | " |
| 50 | i-Propyl | i-Propyl | " | " | " | " | " | " |
| 51 | Phenyl | Phenyl | " | " | " | " | " | " |
| 52 | Et | Propyl | " | " | " | " | " | " |
| 53 | Et | i-Propyl | " | " | " | " | " | " |
| 54 | Propyl | i-Propyl | " | " | " | " | " | " |

7. Herstellung der Verbindungen der Formel (VII) gemäß Verfahren 14

Eine auf 0°C gekühlte Lösung des Didepsipeptids der Formel (IX) (22,9 mmol) und des Didepsipeptids der Formel (VIII) (27,5 mmol) in Dichlormethan (80 ml) wurde mit Diisopropylethylamin (57,3 mmol) und BOP-Cl (29,8 mmol) versetzt, 1 Stunde bei 0°C und 1 Stunde bei Raumtemperatur gerührt. Nach Abfiltrieren des Niederschlages wurde die Lösung mit Dichlormethan verdünnt, dreimal mit wenig Wasser gewaschen, über Natriumsulfat getrocknet und eingeengt. Der Rückstand wurde an Kieselgel mit dem Laufmittel Cyclohexan-Ethylacetat = 15:1 getrennt.

Gemäß dieser Vorschrift wurden die Verbindungen der Formel (VII) erhalten, in welcher die Substituenten die folgende Bedeutung haben:

Tabelle 7

| Nr. | $R^1$ | $R^{12}$ | $R^9$ | $R^8$ | $R^7$ | $R^{10}$ | A | C |
|---|---|---|---|---|---|---|---|---|
| 55 | Et | Et | Me | i-Bu | Bn | i-Bu | H | t-BuO |
| 56 | Propyl | Propyl | " | " | " | " | " | " |
| 57 | i-Propyl | i-Propyl | " | " | " | " | " | " |
| 58 | Phenyl | Phenyl | " | " | " | " | " | " |
| 59 | Et | Propyl | " | " | " | " | " | " |
| 60 | Et | i-Propyl | " | " | " | " | " | " |
| 61 | Propyl | i-Propyl | " | " | " | " | " | " |

8. Herstellung der Verbindungen der Formel (VIII) gemäß Verfahren 17

In eine Lösung des Didepsipeptids der Formel (X) (46,0 mmol) in Dichlormethan (470 mml) wurde 2 Stunden bei 0°C HCl-Gas eingeleitet. Die Reaktionslösung wurde langsam aufgewärmt und über Nacht

bei Raumtemperatur gerührt. Anschließend wurde eingeengt, zweimal mit Dichlormethan (je 150 ml) versetzt, erneut eingeengt und im Hochvakuum getrocknet. Der in Wasser gelöste Rückstand wurde in eine Suspension eines basischen Ionenaustauschers (16,7 g) in 50 ml Wasser eingetropft, 3 Stunden gerührt, abfiltriert und eingeengt. Nach Trocknung im Hochvakuum fiel ein amorphes Pulver an, das ohne weitere Reinigung umgesetzt wurde.

Gemäß dieser Vorschrift wurden die Verbindungen der Formel (VIII) erhalten, in welcher die Substituenten die folgende Bedeutung haben:

Tabelle 8

| Nr. | $R^1$ | $R^9$ | $R^{10}$ | A | Z |
|---|---|---|---|---|---|
| 69 | Et | Me | i-Bu | Bn | OH |
| 70 | Propyl | " | " | " | " |
| 71 | i-Propyl | " | " | " | " |
| 72 | Phenyl | " | " | " | " |

9. Herstellung der Verbindungen der Formel (IX) gemäß Verfahren 18
Eine Lösung des Didepsipeptids der Formel (XI) (60,0 mmol) in 163 ml Ethanol wurde mit 2,91 g Pd-$(OH)_2$/C (20 %) versetzt und 6 Stunden bei Raumtemperatur hydriert. Anschließend wurde filtriert, mit Ethanol nachgewaschen und im Vakuum eingeengt. Der Rückstand wurde an Kieselgel mit dem Laufmittel Cyclohexan-Ethylacetat = 3:1 getrennt.

Gemäß dieser Vorschrift wurden die Verbindungen der Formel (IX) erhalten, in welcher die Substituenten die folgende Bedeutung haben:

Tabelle 9

| Nr. | $R^2$ | $R^8$ | $R^7$ | D | B |
|---|---|---|---|---|---|
| 73 | Et | i-Bu | Bn | H | t-BuO |
| 74 | Propyl | " | " | " | " |
| 75 | i-Propyl | " | " | " | " |
| 76 | Phenyl | " | " | " | " |

10. Herstellung der Verbindungen der Formel (X) gemäß Verfahren 21
Das Caesiumsalz der Formel (XII) wurde in 530 ml Dimethylsulfoxid bei Raumtemperatur vorgelegt und mit der Chlorcarbonsäure der Formel (XIII) (0,212 mol) versetzt. Es wurde 20 Stunden bei Raumtemperatur gerührt, in ges. Kochsalzlösung eingegossen und viermal mit Ethylacetat extrahiert. Die vereinigten organischen Extrakte wurden einmal mit wenig Wasser gewaschen, über Natriumsulfat getrocknet und eingeengt. Die Reinigung des Rückstandes wurde säulenchromatographisch mit dem Laufmittel Cyclohexan-Ethylacetat = 60:1 durchgeführt.

Gemäß dieser Vorschrift wurden die Verbindungen der Formel (X) erhalten, in welcher die Substituenten die folgende Bedeutung haben:

Tabelle 10

| Nr. | $R^1$ | $R^9$ | $R^{10}$ | A | B |
|---|---|---|---|---|---|
| 77 | Et | Me | i-Bu | Bn | t-BuO |
| 78 | Propyl | " | " | " | " |
| 79 | i-Propyl | " | " | " | " |
| 80 | Phenyl | " | " | " | " |

11. Herstellung der Verbindungen der Formel (XI) gemäß Verfahren 22
Die Aminosäure der Formel (XIV) (0,212 mol) wurde in 1000 ml Ethanol und 100 ml Wasser gelöst, mit einer 20 %igen Caesiumcarbonatlösung (200 ml) versetzt und 5 Stunden bei Raumtemperatur gerührt. Anschließend wurde eingeengt, zweimal mit je 250 ml DMF codestilliert und über Nacht bei 80 °C im

Hochvakuum getrocknet. 0,212 mol dieses Caesiumsalzes wurden in 530 ml Dimethylsulfoxid vorgelegt, bei Raumtemperatur mit 0,212 mol der Chlorcarbonsäure der Formel (XV) versetzt und 20 Stunden bei Raumtemperatur gerührt. Die Lösung wurde in gesättigte Kochsalzlösung eingegossen, viermal mit Ethylacetat extrahiert, über Natriumsulfat getrocknet und eingeengt. Der Rückstand wurde säulenchromatographisch mit dem Laufmittel Cyclohexan-Ethylacetat = 100:1 gereinigt.

Analog wurden die Verbindungen der Formel (XI) erhalten, in welcher die Substituenten die folgende Bedeutung haben:

Tabelle 11

| Nr. | $R^2$ | $R^8$ | $R^7$ | A | B |
|---|---|---|---|---|---|
| 81 | Et | i-Bu | Bn | Bn | t-BuO |
| 82 | Propyl | " | " | " | " |
| 83 | i-Propyl | " | " | " | " |
| 84 | Phenyl | " | " | " | " |

## Patentansprüche

**1.** Verbindungen der allgemeinen Formel (I)

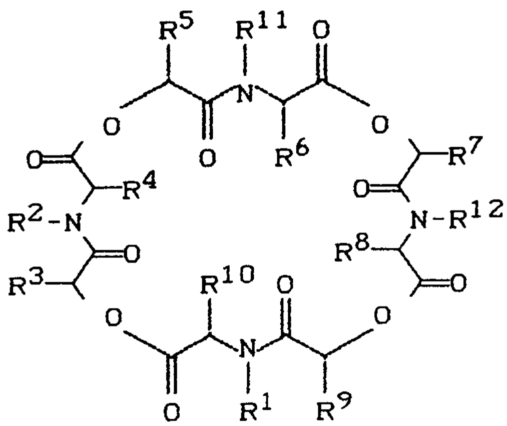

(I)

in welcher

$R^1$ und $R^{12}$ für gleiche oder verschiedene Reste der Gruppe $C_{2-9}$-Alkyl, $C_{1-8}$-Halogenalkyl, $C_{3-6}$-Cycloalkyl, Aralkyl, Aryl stehen,

$R^3$ bis $R^{10}$ für gleiche oder verschiedene Reste der Gruppe Wasserstoff, $C_{1-5}$-Alkyl, das gegebenenfalls durch Hydroxy, Alkoxy, Carboxy, Carboxamid, Imidazolyl, Indolyl, Guanidino, Thio- oder Thioalkyl substituiert sein kann, sowie für gegebenenfalls durch Nitro, oder einen Rest $-NR^{13}R^{14}$, wobei $R^{13}$ und $R^{14}$ unabhängig voneinander für Wasserstoff oder Alkyl stehen oder $R^{13}$ und $R^{14}$ gemeinsam mit dem angrenzenden N-Atom für einen carbocyclischen 5-, 6- oder 7-gliedrigen Ring stehen, der gegebenenfalls auch durch O, S und N unterbrochen sein kann und gegebenenfalls durch $C_1$-$C_4$-Alkyl substituiert ist, oder durch Halogen, Hydroxy, Alkyl oder Alkoxy substituiertes Aryl, Alkylaryl und Hetarylmethyl stehen,

$R^{11}$ und $R^2$ für gleiche oder verschiedene Reste der Gruppe $C_{1-4}$-Alkyl stehen,

sowie deren Stereoisomere.

**2.** Verfahren zur Herstellung der Verbindungen der Formel (I)

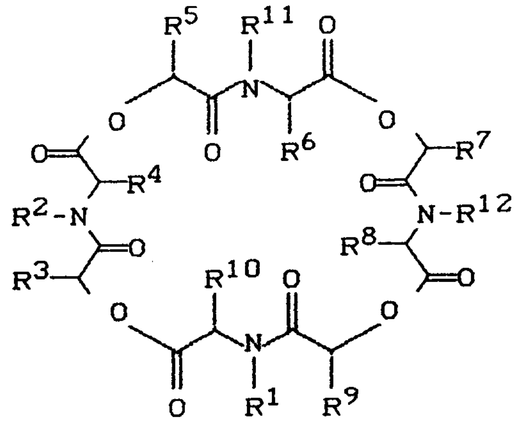

(I)

in welcher

R¹ und R¹² für gleiche oder verschiedene Reste der Gruppe $C_{2-9}$-Alkyl, $C_{1-8}$-Halogenalkyl, $C_{3-6}$-Cycloalkyl, Aralkyl, Aryl stehen,

R³ bis R¹⁰ für gleiche oder verschiedene Reste der Gruppe Wasserstoff, $C_{1-5}$-Alkyl, das gegebenenfalls durch Hydroxy, Alkoxy, Carboxy, Carboxamid, Imidazolyl, Indolyl, Guanidino, Thio- oder Thioalkyl substituiert sein kann, sowie für gegebenenfalls durch Nitro, oder einen Rest $-NR^{13}R^{14}$, wobei $R^{13}$ und $R^{14}$ unabhängig voneinander für Wasserstoff oder Alkyl stehen oder $R^{13}$ und $R^{14}$ gemeinsam mit dem angrenzenden N-Atom für einen carbocyclischen 5-, 6- oder 7-gliedrigen Ring stehen, der gegebenenfalls auch durch O, S und N unterbrochen sein kann und gegebenenfalls durch $C_1$-$C_4$ substituiert ist, oder durch Halogen, Hydroxy, Alkyl oder Alkoxy substituiertes Aryl, Alkylaryl und Hetarylmethyl stehen,

R¹¹ und R² für gleiche oder verschiedene Reste der Gruppe $C_1$-$C_4$-Alkyl stehen,

dadurch gekennzeichnet, daß man offenkettige Octadepsipeptide der Formel (II)

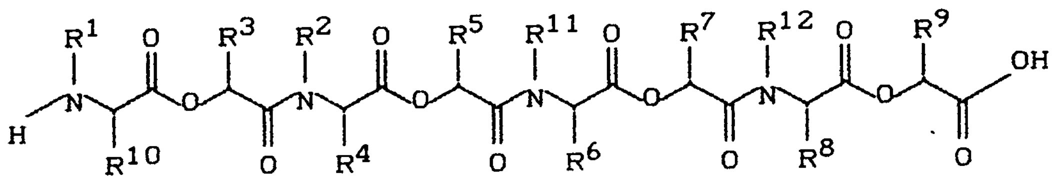

(II)

in welcher

R¹ bis R¹² die oben angegebene Bedeutung haben

in Gegenwart eines Verdünnungsmittels und in Gegenwart eines Kopplungsreagenzes cyclisiert;

**3.** Offenkettige Octadepsipeptide der Formel (II)

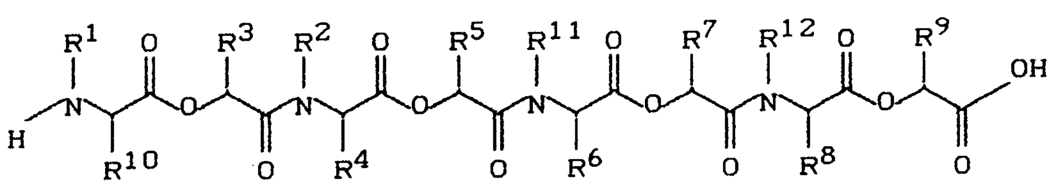

(II)

in welcher

R¹ bis R¹² die in Anspruch 1 angegebene Bedeutung haben.

**4.** Verfahren zur Herstellung der offenkettigen Octadepsipeptide der Formel (II)

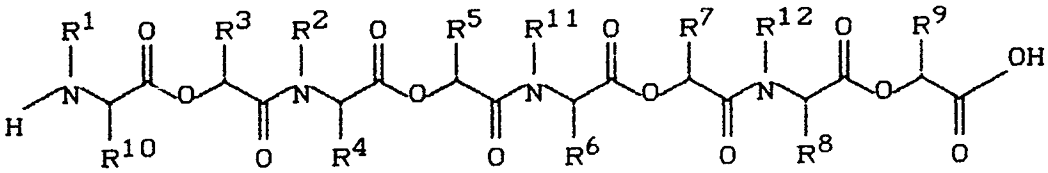

(II)

in welcher
$R^1$ bis $R^{12}$ die in Anspruch 1 angegebene Bedeutung besitzen,
dadurch gekennzeichnet, daß man Verbindungen der Formel (III)

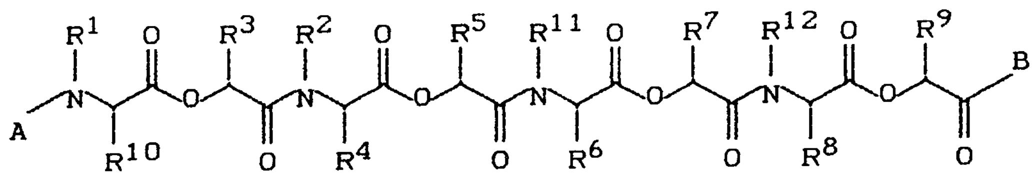

(III)

in welcher
A für Benzyl und
B für OH oder Cl steht und
$R^1$ bis $R^{12}$ die in Anspruch 1 angegebene Bedeutung besitzen,
in Gegenwart eines Verdünnungsmittels und eines Katalysators hydrogenolisiert.

**5.** Verbindungen der Formel (III)

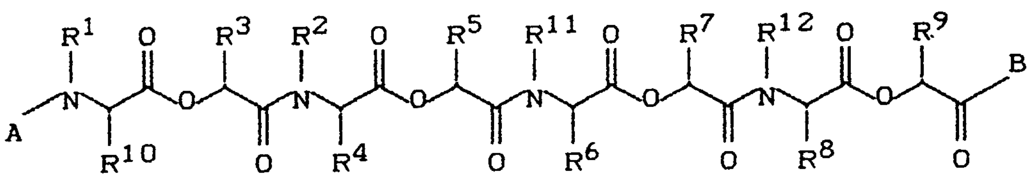

(III)

in welcher
A für Benzyl und
B für OH oder Cl steht und
$R^1$ bis $R^{12}$ die in Anspruch 1 angegebene Bedeutung besitzen.

**6.** Verfahren zur Herstellung der Verbindungen der Formel (III)

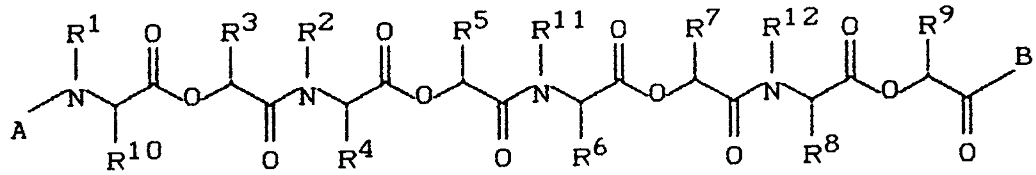

(III)

in welcher

A für Benzyl und

B für OH oder Cl steht und

$R^1$ bis $R^{12}$ die in Anspruch 1 angegebene Bedeutung besitzen,

dadurch gekennzeichnet, daß man Verbindungen der Formel (IV)

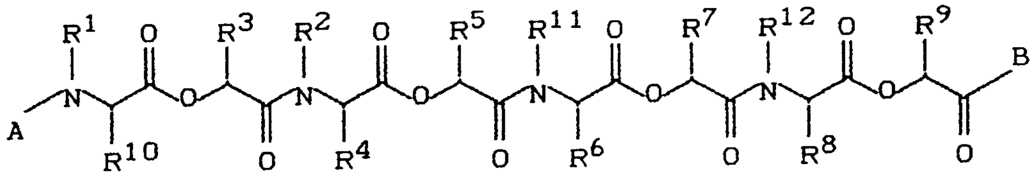

(IV)

in welcher

A für Benzyl und

B für tert.-Butoxy steht sowie

$R^1$ bis $R^{12}$ die in Anspruch 1 angegebene Bedeutung besitzen,

in Gegenwart eines Verdünnungsmittels und einer Protonensäure verseift.

**7.** Verbindungen der Formel (IV)

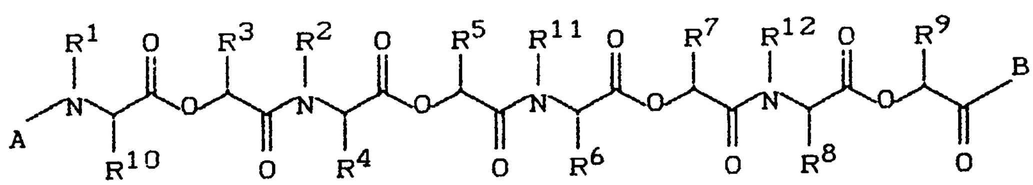

(IV)

in welcher

A für Benzyl und

B für tert.-Butoxy steht und

$R^1$ bis $R^{12}$ die in Anspruch 1 angegebene Bedeutung haben.

**8.** Verfahren zur Herstellung der Verbindungen der Formel (IV)

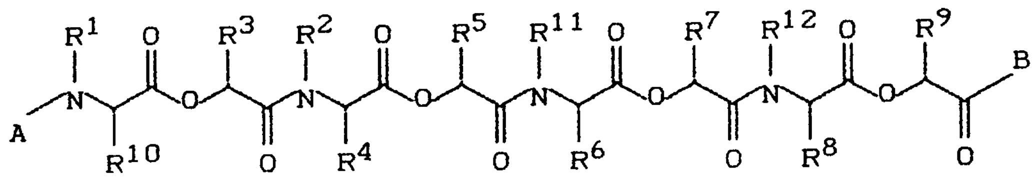

(IV)

in welcher

A für Benzyl und

B für tert.-Butoxy steht und

$R^1$ bis $R^{12}$ die in Anspruch 1 angegebene Bedeutung haben,

dadurch gekennzeichnet, daß man Tetradepsipeptide der Formel (V)

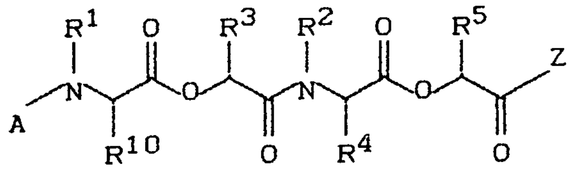

(V)

in welcher

A für Benzyl und

Z für OH oder Cl steht sowie

$R^1$, $R^2$, $R^3$, $R^4$, $R^5$ und $R^{10}$ die in Anspruch 1 angegebene Bedeutung haben

und Tetradepsipeptide der Formel (VI)

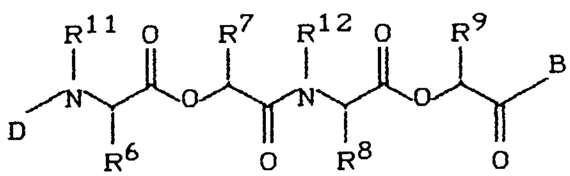

(IV)

in welcher

D für Wasserstoff und

B für tert.-Butoxy steht sowie

$R^6$, $R^7$, $R^8$, $R^9$, $R^{11}$ und $R^{12}$ die in Anspruch 1 angegebene Bedeutung haben,

in Gegenwart eines Verdünnungsmittels und eines geeigneten Kopplungsreagenzes kondensiert.

**9.** Tetradepsipeptide der Formel (V)

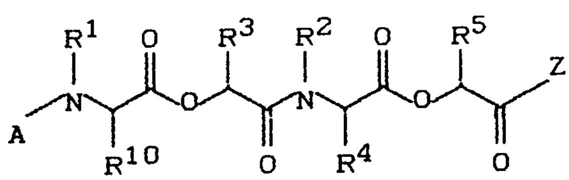

(V)

in welcher

A für Benzyl und

Z für OH oder Cl steht sowie

$R^1$, $R^2$, $R^3$, $R^4$, $R^5$ und $R^{10}$ die in Anspruch 1 angegebene Bedeutung haben.

**10.** Tetradepsipeptide der Formel (VI)

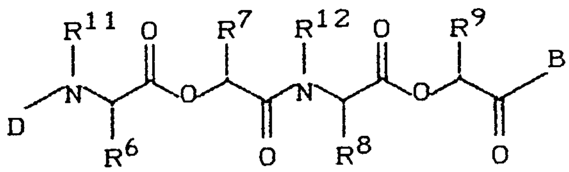

(VI)

in welcher

| | |
|---|---|
| D | für Wasserstoff und |
| B | für tert.-Butoxy steht sowie |
| $R^6$, $R^7$, $R^8$, $R^9$, $R^{11}$ und $R^{12}$ | die in Anspruch 1 angegebene Bedeutung besitzen. |

**11.** Verfahren zur Herstellung der Tetradepsipeptide der Formel (V)

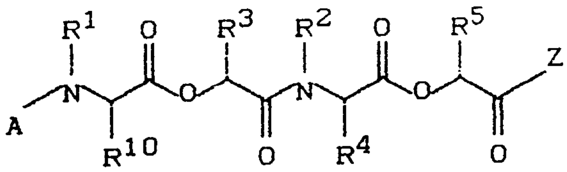

(V)

in welcher

| | |
|---|---|
| A | für Benzyl und |
| Z | für OH oder Cl steht sowie |
| $R^1$, $R^2$, $R^3$, $R^4$, $R^5$ und $R^{10}$ | die in Anspruch 1 angegebene Bedeutung haben, |

dadurch gekennzeichnet, daß man Tetradepsipeptide der Formel (VII)

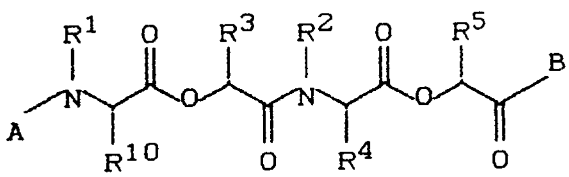

(VII)

in welcher

| | |
|---|---|
| A | für Benzyl und |
| B | für tert.-Butoxy steht sowie |
| $R^1$, $R^2$, $R^3$, $R^4$, $R^5$ und $R^{10}$ | die in Anspruch 1 angegebene Bedeutung haben, |

in Gegenwart eines Verdünnungsmittels und einer Protonensäure verseift.

**12.** Verfahren zur Herstellung der Tetradepsipeptide der Formel (VI)

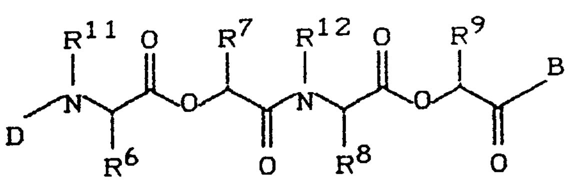

(VI)

in welcher

| | |
|---|---|
| D | für Wasserstoff und |
| B | für tert.-Butoxy steht sowie |
| $R^6$, $R^7$, $R^8$, $R^9$, $R^{11}$ und $R^{12}$ | die in Anspruch 1 angegebene Bedeutung haben, |

dadurch gekennzeichnet, daß man Tetradepsipeptide der Formel (VII)

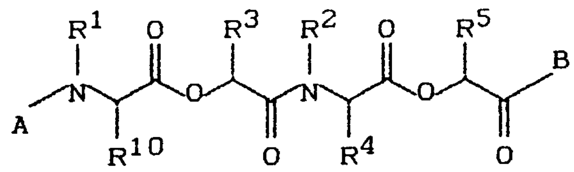

(VII)

in welcher
A für Benzyl und
B für tert.-Butoxy steht sowie
$R^1$, $R^2$, $R^3$, $R^4$, $R^5$ und $R^{10}$ die in Anspruch 1 angegebene Bedeutung besitzen,
in Gegenwart eines Verdünnungsmittels und eines Katalysators hydrogenolysiert.

**13.** Tetradepsipeptide der Formel (VII)

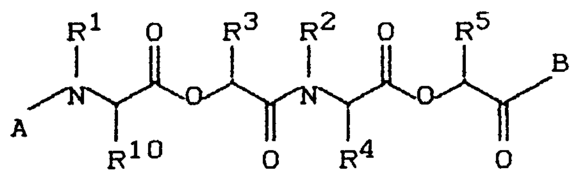

(VII)

in welcher
A für Benzyl und
B für tert.-Butoxy steht sowie
$R^1$, $R^2$, $R^3$, $R^4$, $R^5$ und $R^{10}$ die in Anspruch 1 angegebene Bedeutung haben.

**14.** Verfahren zur Herstellung der Tetradepsipeptide der Formel (VII)

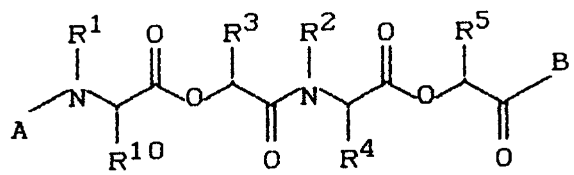

(VII)

in welcher
A für Benzyl und
B für tert.-Butoxy steht sowie
$R^1$, $R^2$, $R^3$, $R^4$, $R^5$ und $R^{10}$ die in Anspruch 1 angegebene Bedeutung besitzen,
dadurch gekennzeichnet, daß man Didepsipeptide der Formel (VIII)

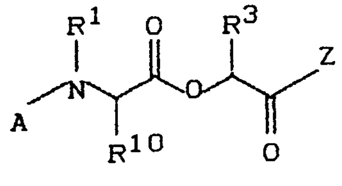

(VIII)

in welcher
A für Benzyl und
Z für OH oder Cl steht sowie
$R^1$, $R^3$ und $R^{10}$ die in Anspruch 1 angegebene Bedeutung besitzen und
Didepsipeptide der Formel (IX)

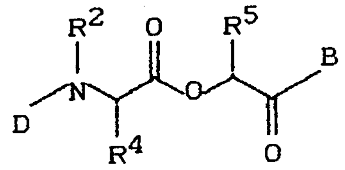

(IX)

in welcher

D für Wasserstoff und
B für tert.-Butoxy steht sowie
$R^2$, $R^4$ und $R^5$ die in Anspruch 1 angegebene Bedeutung besitzen,

in einem Verdünnungsmittel in Gegenwart eines geeigneten Kopplungsreagenzes kondensiert.

**15.** Didepsipeptide der Formel (VIII)

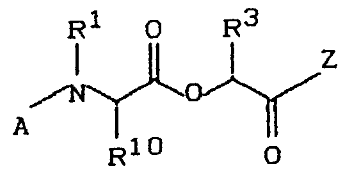

(VIII)

in welcher

A für Benzyl und
Z für OH oder Cl steht sowie
$R^1$, $R^3$ und $R^{10}$ die in Anspruch 1 angegebene Bedeutung besitzen.

**16.** Didepsipeptide der Formel

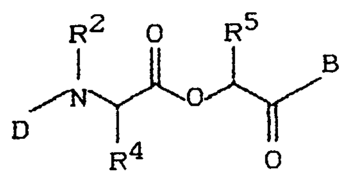

(IX)

in welcher

D für Wasserstoff und
B für tert.-Butoxy steht sowie
$R^2$, $R^4$ und $R^5$ die in Anspruch 1 angegebene Bedeutung besitzen.

**17.** Verfahren zur Herstellung von Didepsipeptiden der Formel (VIII)

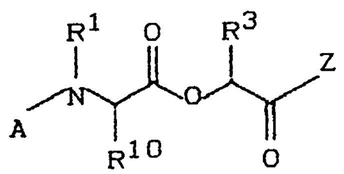

(VIII)

in welcher

Z für OH oder Cl und
A für Benzyl steht sowie

$R^1$, $R^3$ und $R^{10}$ die in Anspruch 1 angegebene Bedeutung besitzen. Die Verfahren sind dadurch gekennzeichnet, daß man eine Verbindung der Formel (X)

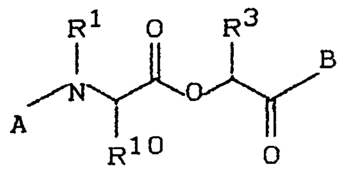

(X)

in welcher

| | |
|---|---|
| A | für Benzyl steht und |
| B | für tert.-Butoxy sowie |
| $R^1$, $R^3$ und $R^{10}$ | die in Ansprch 1 angegebene Bedeutung besitzen |

und einem Verdünnungsmittel in Gegenwart einer Protonensäure verseift und gegebenenfals in das Säurechlorid überführt.

**18.** Verfahren zur Herstellung von Didepsipeptiden der Formel (IX)

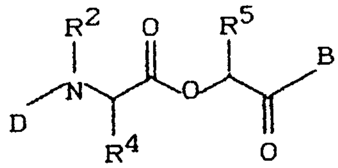

(IX)

in welcher

| | |
|---|---|
| D | für Wasserstoff und |
| B | für tert.-Butoxy steht sowie |
| $R^2$, $R^4$ und $R^5$ | die in Anspruch 1 angegebene Bedeutung besitzen, |

dadurch gekennzeichnet, daß man Verbindungen der Formel (XI)

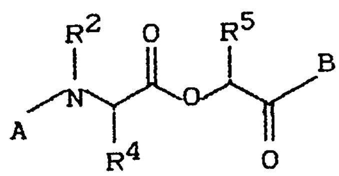

(XI)

in welcher

| | |
|---|---|
| A | für Benzyl und |
| B | für tert.-Butoxy steht sowie |
| $R^2$, $R^4$ und $R^5$ | die in Anspruch 1 angegebene Bedeutung besitzen, |

in Gegenwart eines Verdünnungsmittels und eines Katalysators hydrogenolysiert.

**19.** Didepsipeptide der Formel (X)

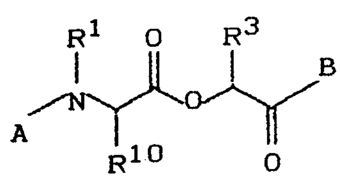

(X)

in welcher

| | |
|---|---|
| A | für Benzyl steht und |
| B | für tert.-Butoxy sowie |
| $R^1$, $R^3$ und $R^{10}$ | die in Anspruch 1 angegebene Bedeutung besitzen. |

**20.** Didepsipeptide der Formel (XI)

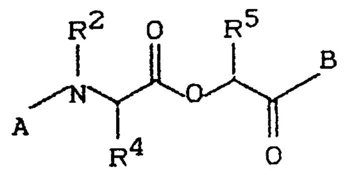

(XI)

in welcher
A für Benzyl und
B für tert.-Butoxy steht sowie
$R^2$, $R^4$ und $R^5$ die in Anspruch 1 angegebene Bedeutung besitzen.

**21.** Verfahren zur Herstellung von Didepsipeptiden der Formel (X)

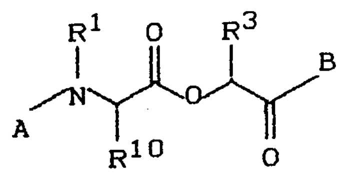

(X)

in welcher
A für Benzyl und
B für tert.-Butoxy steht sowie
$R^1$, $R^3$ und $R^{10}$ die in Anspruch 1 angegebene Bedeutung besitzen,
dadurch gekennzeichnet, daß man eine Aminocarbonsäure der Formel (XII)

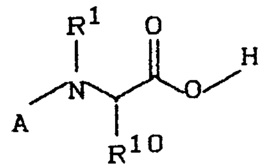

(XII)

in welcher
A für Benzyl und
$R^1$ und $R^{10}$ die in Anspruch 1 angegebene Bedeutung besitzen,
in Form ihres Alkalimetallsalzes und eine $\alpha$-Halogencarbonsäure der Formel (XIII)

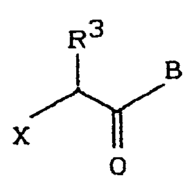

(XIII)

in welcher
X für Cl oder Br und
B für tert.-Butoxy steht sowie
$R^3$ die in Anspruch 1 angegebene Bedeutung besitzt,
in Gegenwart eines Verdünnungsmittels koppelt.

**22.** Verfahren zur Herstellung von Didepsipeptiden der Formel (XI)

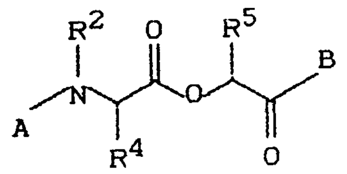

(XI)

in welcher

A für Benzyl und

B für tert.-Butoxy steht sowie

$R^2$, $R^4$ und $R^5$ die in Anspruch 1 angegebene Bedeutung besitzen,

dadurch gekennzeichnet, daß man eine Aminocarbonsäure der Formel (XIV)

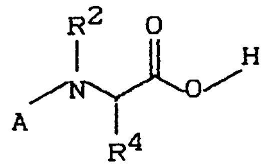

(XIV)

in welcher

A für Benzyl und

$R^1$ und $R^{10}$ die in Anspruch 1 angegebene Bedeutung besitzen,

in Form ihres Alkalimetallsalzes und eine α-Halogencarbonsäure der Formel (XV)

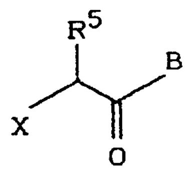

(XV)

in welcher

X für Cl oder Br und

B für tert.-Butoxy steht sowie

$R^5$ die in Anspruch 1 angegebene Bedeutung besitzt,

in Gegenwart eines Verdünnungsmittels koppelt.

**23.** Verwendung von cyclischen Depsipeptiden der Formel (I) gemäß Anspruch 1 zur Herstellung von endoparasitiziden Mitteln.

**24.** Verwendung von cyclischen Depsipeptiden der Formel (I) gemäß Anspruch 1 als Endoparasitizide.

**25.** Endoparasitizide Mittel gekennzeichnet durch einen Gehalt an mindestens einem cyclischen Depsipeptid der Formel (I) gemäß Anspruch 1.

Europäisches Patentamt

# EUROPÄISCHER RECHERCHENBERICHT

Nummer der Anmeldung
EP 94 10 7544

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | TETRAHEDRON LETTERS.<br>Bd. 26, Nr. 43 , 1985 , OXFORD GB<br>Seiten 5257 - 5260<br>H.-G. LERCHEN ET AL 'Stereoselective Synthese von D-alpha-Hydroxycarbonsäuren bzw. D-alpha-Hydroxycarbonsäuren enthaltenden Depsipeptiden aus L-Aminosäuren'<br>* Seite 5258; Tabelle 1 *<br>--- | 16,20 | C07D273/00<br>A61K31/395<br>C07K11/00<br>C07K11/02 |
| X | CHEMICAL ABSTRACTS, vol. 69, no. 23, 2. Dezember 1968, Columbus, Ohio, US; abstract no. 97135,<br>I. I. MIKHALEVA ET AL 'Relation between structure and biological action in a series of enniatin B and its analogs'<br>Seite 9115 ;<br>* Zusammenfassung *<br>& ZH. OBSHCH. KHIM.<br>Bd. 38, Nr. 6 , 1968<br>Seiten 1228 - 1239<br>--- | 16 | RECHERCHIERTE SACHGEBIETE (Int.Cl.5) |
| D,A | EP-A-0 382 173 (MEIJI SEIKA KAISHA)<br>* Ansprüche 1,5 *<br>--- | 1,25 | C07D<br>A61K<br>C07K |
| A | CHEMICAL ABSTRACTS, vol. 94, no. 13, 30. März 1981, Columbus, Ohio, US; abstract no. 103802,<br>AKIRA ISOGAI ET AL 'Bassianolide: syntheses of its analogs and NMR studies'<br>Seite 792 ;<br>* Zusammenfassung *<br>& PEPTIDE CHEMISTRY, 16TH 1978<br>Seiten 165 - 170<br>----- | 1 | |

Der vorliegende Recherchenbericht wurde für alle Patentansprüche erstellt

| Recherchenort | Abschlußdatum der Recherche | Prüfer |
|---|---|---|
| DEN HAAG | 10. August 1994 | Voyiazoglou, D |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument